(19) Europäisches Patentamt
European Patent Office
Office européen des brevets

(11) Publication number: 0 002 228
B1

(12) EUROPEAN PATENT SPECIFICATION

(45) Date of publication of patent specification: 29.02.84

(21) Application number: 78101426.1

(22) Date of filing: 21.11.78

(51) Int. Cl.³: C 07 C 50/32, C 07 C 46/00, C 07 C 91/10, A 61 K 31/12

# (54) Hydroxy naphthoquinone derivatives, their preparations for treating and preventing theileriosis in cattle and sheep, processes for their synthesis; naphthoquinone derivatives.

(30) Priority: 22.11.77 GB 4848677

(43) Date of publication of application: 13.06.79 Bulletin 79/12

(45) Publication of the grant of the patent: 29.02.84 Bulletin 84/9

(84) Designated Contracting States: BE CH DE FR GB NL

(56) References cited:
DE - A - 2 705 599
FR - A - 2 323 676
US - A - 2 553 648
US - A - 3 367 830

The file contains technical information submitted after the application was filed and not included in this specification

(73) Proprietor: THE WELLCOME FOUNDATION LIMITED
183-193 Euston Road
London NW1 2BP (GB)

(72) Inventor: McHardy, Nicholas
10 Downs Road
Beckenham Kent (GB)
Inventor: Hudson, Alan Thomas
"Lustleigh" Stonehouse Lane
Halstead Sevenoaks Kent (GB)

(74) Representative: Berg, Wilhelm, Dr. et al,
Dr. Berg, Dipl.-Ing. Stapf, Dipl.-Ing. Schwabe, Dr. Dr. Sandmair Patentanwälte Postfach 860245
D-8000 München 86 (DE)

(56) References cited:
JOURNAL OF THE AMERICAN CHEMICAL SOCIETY, vol. 70, October 1948, pages 3165-3174 L. FIESER: "Naphthoquinone Antimalarials. III. Diene Synthesis of 1,4-Naphthoquinones"
Journal of the American Chemical Society, volume 70, pages 3150-3164, 1948

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Hydroxy naphthoquinone derivatives, their preparations for treating and preventing theileriosis in cattle and sheep, processes for their synthesis; naphthoquinone derivatives

The present invention relates to compounds and preparations for treating and preventing theileriosis in cattle and sheep, and to processes for the synthesis of such compounds.

Theileriosis is a disease caused by protozoa of the genus *Theileria*. In cattle, *T.parva*, *T.lawrencei*, *T.annulata* and *T.mutans* are responsible for substantial losses, mainly in Central and East Africa, and the Middle East. In sheep, *T.hirci* and *T.ovis* are the causative agents and the disease is prevalent in the Middle East. Infected ticks transmit these protozoa to the mammalian host. The lymphocyte cells are infected which then proceed to divide rapidly contrary to their normal function. Death is caused by the release of toxic products from rupturing lymphocytes in addition to other harmful effects attributed to the parasites themselves. After release from the lymphocytes, the *Theileria* parasite infects the erythrocytes and then the ticks, which feed on the infected animals.

In the field there is no known effective treatment of theileriosis. The only drug that has hitherto been used for the treatment of cattle and sheep is oxytetracycline, the usual use of which is against anaplasmosis and bacterial infections. According to S. F. Barnett in Infectious Blood Diseases of Man and Animals, Vol. III, Eds. Weinman D. & Ristic M., Academic Press 1968, this compound has had only very limited success when given before an infection is established, but no effect once the infection is actually established. Frequently, the symptoms of anaplasmosis are misinterpreted and theileriosis is diagnosed, and as a result oxytetracycline is administered, often in massive doses up to nearly the toxic level. Apparent recovery from theileriosis is in actual fact real recovery from anaplasmosis.

DE—A—2 705 599 describes 2-hydroxy-3-cyclohexylalkyl- and 2-hydroxy-3-cyclohexyl-1, 4-naphthoquinone derivatives as effective for the prevention and treatment of theileriosis, and particularly against infections with *T.annulata* and *T.parva*. The former type of compounds could be prepared from the corresponding 2-unsubstituted intermediate by oxidation and subsequent hydrolysis. However, when the cyclohexyl group is directly linked with the naphthoquinone ring system, it has been suggested that 2-hydroxy-1,4-naphthoquinone should be reacted with hexahydrobenzoylperoxide according to a method described in the literature (c.f. U.S.P. 2553647), though only a poor yield has been obtained by this route.

It has now been found that differently substituted 1,4-naphthoquinone derivatives show high and advantageous activity against various theileria species in tests under *in vitro* or *in vivo* conditions. This enables the preparation of novel therapeutic compositions and the treatment of animals against the diseases caused by these organisms. It has also been found that the compounds have activity against sheep scab *in vivo* tests.

According to one aspect of the invention, therefore, there is provided a 2-hydroxy-1,4-naphthoquinone derivative of the general formula (II), a tautomer or a pharmaceutically acceptable salt thereof,

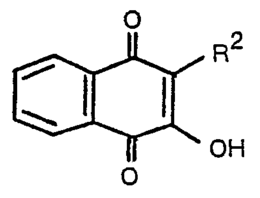

(II)

wherein $R^2$ is an unsubstituted monocyclic cycloalkyl group having 7 to 12 carbon atoms. In particular the cycloalkyl ring preferably has 7 to 9 carbon atoms.

Tautomerism may cause the 3-hydroxy group to donate its acidic hydrogen to the adjacent oxo group, but it is believed that formula (II) represents the more stable state. Since the hydroxy group in the formula may form salts with appropriate bases, the pharmaceutically acceptable salts of the compounds include those with an alkali metal cation, such as sodium or potassium, and those with organic bases such as N-methyl glutamine.

The most preferred compounds of formula (II) are 2-cycloheptyl-3-hydroxy-1,4-naphthoquinone and 2-cyclooctyl-3-hydroxy-1,4-naphthoquinone.

The invention provides, in a further aspect, a therapeutic composition comprising, as active ingredient an effective dosage of a 2-hydroxy-1,4-naphthoquinone of formula (II) as herein defined or a tautomer or pharmaceutically acceptable salt thereof.

The therapeutic preparations comprising a compound of formula (II), may take the form of shaped tablets or a composition with a pharmaceutically acceptable carriers, including sealed containers, or ingredients such as excipients. Advantageously the preparation may be presented in a unit dosage form, or as a sterile, sealed formulation.

The "effective amount, dosage or unit-dosage" as used herein is denoted to mean a predetermined amount of the compound which is sufficiently effective against *Theileria* organisms, for instance in cattle or sheep when administered *in vivo*.

To contribute to or achieve prophylaxis or cure, some preparations may contain multiples of the dosage required by a single animal.

A typical initial dose for cattle weighing 400 kg may be 1 to 10 g conveniently 2 to 5 g, and for calves or sheep 250 mg to 2.5 g, or preferably about half to one gramme of the active compound, but further dosages may contain 50 to 500 mg for an animal.

Preparations comprising a compound of formula (II), may be presented with a suitable carrier in formulations for parenteral (subcutaneous or preferably intramuscular), intravenous or oral administration. A sterile injectable formulation is advantageously formed in an organic carrier, which may also contain bacteriostatic agents, antioxidants, buffer solutes to render the preparation isotonic with the blood, thickening agents, suspending agents or other pharmaceutically acceptable additives.

The injectable formulations may be presented in unit dose containers, such as ampoules, or disposable injection devices, or in multi-dose forms such as bottles from which an appropriate dose may be withdrawn.

The formulations for oral administration may include as carriers solids to form tablets, pastes, granules or powder, or may include liquids for suspensions or solutions, which may contain diluents, binding agents, dispersing agents, surface active agents, lubricating agents, coating materials, colouring agents, solvents, thickening agents, suspending agents or other pharmaceutically acceptable additives, and these preparations may be presented in unit dose form or multi-dose form, or as additives to foodstuffs. The compounds of formula (II) may also be formulated into a salt-lick so that the animals can obtain prophylactic treatment when in the field.

Compounds of formula (II) can also be formulated into a pour-on preparation containing for example up to 10% by weight active compound in a suitable carrier such as dimethyl sulphoxide.

The therapeutic composition may be in the form of a package together with instructions as to the usage of the same to achieve protection against or cure from theileriosis in appropriate mammals.

For therapeutic treatment the compound of formula (II), or a salt thereof may be administered as one relatively large dose on the day the temperature of the animal rises and schizonts appear or later in the disease syndrome than this, followed by smaller daily doses for the next few days, for example 5 days. The total dosage over the treatment period is preferably from 1 to 200 mg/kg active ingredient, more preferably 2 to 60 mg/kg active ingredient, most preferably 5 to 25 mg/kg. The treatment may alternatively comprise a single dose or 2 doses administered on consecutive days, or could comprise up to a total of 10 doses. For instance, an effective single dose administered to a 200 kg cow may comprise 1000 mg which is given on day 1 followed by a daily dose of 200 mg given on five consecutive days.

In prophylactic treatment, for example when an animal is suspected of having been exposed to infection, the compound of formula (II) may be administered for instance as a dose of 2.5 mg/kg on the first day, followed by consecutive daily doses of 0.5 mg/kg for up to nine days, giving a total dose of 7 mg/kg. Depending on the severity of the risk or exposure, daily doses of 0.2 to 5 mg/kg may be administered. The duration of such preventive measure may last from 4 to 20 or even 120 days. Alternatively, the compound can be incorporated into a slow release chronic implant, which is formulated into a pellet with a relatively insoluble carrier, so that it can be injected under the skin by means of a gun. As the pellet dissolves the active ingredient is released slowly over a period of for example 4 months, so that a low level of protection is maintained, which is equivalent to the above regimen.

The compounds of formula (II) may be employed in a method for treating theileriosis in cattle or sheep, either prophylactically or therapeutically or both, comprising the administration of an effective amount of a compound of formula (II), or a pharmaceutically acceptable salt thereof, to an animal infected or potentially exposed to an infection, with a pathogenic *Theileria* species. In particular; the method of treatment can be effected by the use of a preparation or composition of such compounds, as hereinbefore defined.

The treatment as hereinbefore defined includes the administration of a vaccine, containing living *Theileria* organisms of one or more species or strains acting as effective antigens. Such vaccines for the purpose may for instance be prepared from a suspension of ground ticks, which have been infected by *Theileria* species, e.g. *T.parva* and *T.lawrencei* (c.f. Radley. *D*.E. *et al*. (1975), Veterinary Parasitology, 51—60) or other infected material, e.g. cultured mammalian cells infected with *Theileria* or cultured tick cells or salivary tissue or their products or exudates infected with *Theileria*. Conveniently, a dosage of an active compound hereinbefore described, representing 1 to 20 mg/kg of the host animal, is administered on the day of vaccination or up to 7 or even 14 days after vaccination. Thus the initial treatment can consist of the administrations of a combination product which provides for an immediate release of the antigen, and a later release of the active compound delayed for seven or more days. The initial dosage may be followed by daily dosages of 1 to 10 mg/kg for up to 10 days.

Compounds of formula (II) can be prepared by converting the corresponding 3-halogeno-, e.g. 3-chloro- or 3-bromo, analogues into the 3-hydroxy-substituted compounds by alkaline hydrolysis for instance with an alkali metal hydroxide in a suitable medium. For instance potassium hydroxide in aqueous methanolic medium has been found convenient. The starting 3-halogeno derivative may be obtained by oxidation and subsequent halogenation from the corresponding 4a, 5, 7,7a-tetrahydro-2-cycloalkyl-1,4-naphthoquinone according to the method suggested by Fieser, L., (*J. Am. Chem. Soc.*, 1948, 3165) originally starting from a substituted or unsubstituted 2-cycloalkyl phenol.

The 2-cycloalkyl substituted 1,4-naphthoquinone derivatives can be prepared with advantage by

reacting an appropriately 3-substituted 1,4-naphthoquinone with a donor compound providing the cycloalkyl substituent in a free radical under oxidising conditions. The latter may be provided by an appropriate oxidising agent or by a peroxide structure of the donor itself.

Accordingly the present invention provides in a yet further aspect a process for the preparation of a compound of formula (II) which comprises preparation of a 1,4-naphthoquinone derivative of formula (III)

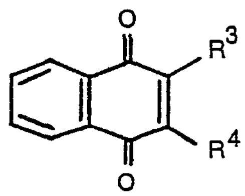

(III)

wherein $R^3$ is an unsubstituted monocyclic cycloalkyl group having 7 to 12 carbon atoms, and $R^4$ is a halogeno, e.g. chloro or bromo atom, or a hydroxy, acetoxy, or alkoxy group; by reacting a compound of formula (IV)

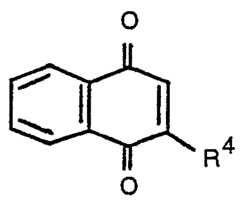

(IV)

wherein $R^4$ is as hereinbefore defined; with a cycloalkyl donor compound containing the $R^3$ moiety as hereinbefore defined in a form capable of providing the cycloalkyl group as a free radical either spontaneously or under oxidising conditions and, where appropriate, hydrolysing the $R^4$ group other than hydroxy into a 3-hydroxy group in the resulting 2-cycloalkyl substituted condensate.

The relevant cycloalkanes may themselves act as a donor when employed in combination with a peroxide type of agent, such as di-*t*-butylperoxide. Usually the liquid or molten cycloalkane itself acts as a solvent or medium for the reaction, which is normally carried out at elevated temperatures, e.g. above 100°C (c.f. Hoyser, E.S. *et. al., J. Org. Chem.*, 1968, *33* (2), 576).

An alternative donor can be a corresponding cycloalkane carboxylic acid which may undergo oxidative decarboxylation. For instance persulphate with a catalyst, such as silver ions, is convenient for the purpose. (c.f. Jacobson N. *et al., Annalen*, 1972, 763, 135 and *Acta Chem. Scand*, 1973, *27*, 3211). Preferably, when persulphate is used under those conditions, the reaction is carried out with a 1,4-naphthoquinone substituted for $R^4$ with a group other than hydroxy. Conveniently ammonium persulphate can be used as the oxidising agent, and the catalyst is silver nitrate. Hydrolysis subsequent to the main coupling reaction, may if required, provide the hydroxy group. Alkaline conditions are usually preferred for the hydrolysis.

An example for the donor carrying itself a peroxide grouping is the method employing an appropriately substituted cycloalkanoyl peroxide as suggested by U.S.P. 2 553 647.

The compounds according to formula (II) may also be prepared from the next Regler homologue 2-hydroxy-3-cycloalkylmethyl-1,4-naphthoquinone by Hooker oxidation (c.f. *J. Am. Chem. Soc.*, 1948, 3174 or 3215).

The provision of the cycloalkyl free radical by a spontaneous release from the donor can for instance be achieved by the use of a tricycloalkylborane. Such reagent can easily be prepared by reacting the cycloalkene with borane dimethylsulphide. Conveniently the reaction is carried out in a solvent such as tetrahydro-furan.

The 2-substituted-1,4-naphthoquinones required for some of the above reaction schemes, are available by synthetic processes known in themselves from the literature, e.g. Fieser, L., *J. Am. Chem. Soc.*, 1948, 3165, or prepared accordingly by analogous techniques.

The following Examples illustrate the invention:—

Example 1

Cycloheptane (54.98 g), 2-hydroxy-1,4-naphthoquinone (1.3 g), and di-*t*-butylperoxide (3.5 g) were heated at 125°C for 7 hours. After overnight cooling to room temperature the mixture was transferred (using toluene when necessary) to a round bottomed flask and the cycloheptane removed *in vacuo*. The residue was extracted with hot benzene (3 x 100 ml) which was filtered to remove unreacted 2-hydroxy-1,4-naphthoquinone. Evaporation of the filtrate gave an oily solid (1.65 g) which was further extracted with hot petrol (b.p. 60—80°C, 3 x 15 ml). The petrol extracts were combined, filtered to remove more unreacted 2-hydroxy-1,4-naphthoquinone (420 mg) and evaporated to give crude product as a slightly sticky solid (1.23 g, 60.7% yield). Recrystallisation from petrol (b.p.

60—80°C, 7 ml), after seeding with authentic product and scratching, yielded a solid 960 mg (47.5% yield), m.p. 95—6°C of 3-cycloheptyl-2-hydroxy-1,4-naphthoquinone.

Example 2

2-Chloro-1,4-naphthoquinone (19.2 g) was mixed with cycloheptanecarboxylic acid (14.2 g) and silver nitrate (5.0 g) in acetonitrile (170 ml) and refluxed with stirring. Ammonium persulphate (60 g) in water (250 ml) was then added dropwise over 1 hour and the resulting mixture refluxed for a further hour, cooled in ice and filtered. The solid was washed with hot chloroform (3 × 75 ml) and discarded (mainly inorganic salts). The chloroform wash was dried with magnesium sulphate and evaporated to give the crude product as an oil (16.41 g), which on standing semi-solidified. On trituration with ethyl acetate (3 ml)/ethanol (3 ml) solid (5.11 g) was obtained m.p. 88—95°C, which on re-crystallisation from isopropanol (20 ml) gave product 2-chloro-3-cycloheptyl-1,4-naphthoquinone, 3.63 g, m.p. 94—5°C.

Evaporation of the filtrate gave an oil which was dissolved in toluene and columned on silica gel. Elution with toluene gave 9.2 g crude product which could be further purified by recrystallisation from methanol to yield pure product, m.p. 97—98°C.

Total crude yield of product was 45%.

Example 3

2-Chloro-3-cycloheptyl-1,4-naphthoquinone (6.35 g) was dissolved in methanol (160 ml), heated to reflux and a solution of potassium hydroxide (6.38 g) in water (64 ml) added at such a rate to maintain reflux. When all this had been added the solution was refluxed for an additional 15 minutes and then (if reaction complete as shown by thin layer chromatography (TLC) of acidified extract with toluene), cooled in ice and acidified (conc. HCl). The resulting precipitated oil solidified on scratching and standing. Filtration of solid gave 4.23 g (70% yield) of orange-yellow material m.p. 88—90°C. Purification by recrystallisation from petrol (b.p. 60—80°C) (25 ml) afforded 3.17 g, m.p. 94—6°C of 3-cycloheptyl-2-hydroxy-1,4-naphthoquinone.

Example 4

Cyclooctane was used in the manner and under the conditions described in Example 1 to provide 3-cyclooctyl-2-hydroxy-1,4-naphthoquinone, m.p. 74—75°C.

Example 5

Cyclododecane was used in the manner and under the conditions described in Example 1 to provide 3-cyclododecyl-2-hydroxy-1,4-naphthoquinone, m.p. 155—156°C.

Example 6

A "pour-on" formulation for cattle was prepared as follows:—

| | |
|---|---|
| 2-Hydroxy-3-cycloheptyl-1,4-naphthoquinone | 4 parts by weight |
| Dimethyl sulphoxide | 10 parts by weight |
| Castor oil | to 100 parts by weight |

Example 7

An aqueous suspension was prepared as follows:—

| | |
|---|---|
| 2-Hydroxy-3-cycloheptyl-1,4-naphthoquinone | 1.00 parts by weight |
| Neosyl® | 16.00 parts by weight |
| Bentonite | 3.20 parts by weight |
| Glycerin | 15.00 parts by weight |
| Sodium benzoate | 1.00 parts by weight |
| Bevaloid 35/2® | 1.00 parts by weight |
| Thymol | 0.04 parts by weight |
| Water | 62.76 parts by weight |
| | 100.00 |

Example 8

A salt block was prepared by mixing the finely divided 2-hydroxy-3-cycloheptyl-1,4-naphthoquinone (0.5 parts by weight) with sodium chloride (99.5 parts by weight) and the mixture was pressed into blocks.

Example 9

The following paste was prepared:—

| | |
|---|---|
| 2-Hydroxy-3-cycloheptyl-1,4-naphthoquinone | 3.0 parts by weight |
| Gum tragacanth | 4.0 parts by weight |
| Bevaloid 35/3® | 1.0 parts by weight |
| Nipagin "M"® | 0.1 parts by weight |
| Glycerin | 19.0 parts by weight |
| Water | 72.9 parts by weight |
| | 100.0 |

Example 10

A solution for subcutaneous injection was prepared by mixing:—

| | |
|---|---|
| 2-Hydroxy-3-cycloheptyl-1,4-naphthoquinone | 4.5 parts by weight |
| Methocel® | 2.0 parts by weight |
| Nipagin "M"® | 0.1 parts by weight |
| Water | 93.4 parts by weight |
| | 100.0 |

Example 11

A solution for intramuscular injection was prepared by mixing:—

| | |
|---|---|
| 2-Hydroxy-3-cycloheptyl-1,4-naphthoquinone | 9.5 parts by weight |
| Dimethyl sulphoxide | 19.0 parts by weight |
| Sorbitan monooleate | 4.5 parts by weight |
| Corn oil | 67.0 parts by weight |
| | 100.0 |

Example 12

Formulations were prepared, as in Examples 6 to 11 except that the active ingredient was 2-hydroxy-3-cyclooctyl-1,4-naphthoquinone.

Example 13

Cultures of the bovine lymphoblastoid cells infected with the macroschisont stage of *T.parva* were incubated for 48 hours in the presence of various concentrations of 2-hydroxy-3-cycloheptyl-1,4-naphthoquinone. Other cultures were incubated without the drug, to act as controls. Subsequently smears were prepared in order to ascertain the proportion of cells which contained the parasite. The results were as follows:—

| Compound | Concentration mg/litre | % cells infected with *T.parva* after 48 hrs incubation |
|---|---|---|
| Control | — | 98 |
| I,$R^2$ = cycloheptyl | 1.0 | 17 |
| | 0.1 | 13 |
| | 0.01 | 21 |
| | 0.001 | 46 |
| | 0.0001 | 98 |
| Oxytetracycline | 100.0 | 97 |

It was apparent that the tested compound had showed a high level of activity, compared with that of oxytetracycline or the state of the controls. This was taken as an indication of *in vivo* activity (c.f. McHardy, N. *et al, Nature* (*London*), 1976, *261*, 698).

## Claims

1. A compound of formula (II)

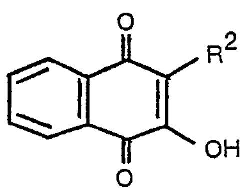

(II)

a tautomer or a pharmaceutically acceptable salt thereof wherein $R^2$ an unsubstituted monocyclic cycloalkyl group having 7 to 12 carbon atoms.

2. A compound as claimed in claim 1 wherein $R^2$ has 7 or 8 carbon atoms.

3. A compound as claimed in claim 1 or claim 2 in the form of a sodium, potassium or N-methyl glucamine salt.

4. 2-Cycloheptyl-3-hydroxy-1, 4-naphthoquinone.

5. 2-Cyclooctyl-3-hydroxy-1, 4-naphthoquinone.

6. A compound of formula (II) as defined in any one of claims 1 to 5 for use in veterinary medicine.

7. A process for producing a compound of formula (II) as defined in claim 1, characterised in that the corresponding 3-halogeno analogue of the compound is hydrolysed under alkaline conditions to provide the 3-hydroxy-2-substituted compound.

8. A process for producing a compound of formula (II), as defined in claim 1, characterised in that a compound of formula (IV)

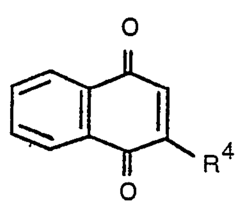

(IV)

wherein $R^4$ is a halogeno, hydroxy, acetoxy or alkoxy group is reacted with a cycloalkyl donor compound corresponding with the desired cycloalkyl group $R^2$, and when $R^4$ is other than hydroxy, hydrolysing the resultant 3-halogeno- or 3-acetoxy- or 3-alkoxy- compound under alkaline conditions.

9. A process for producing a compound of formula (II), as defined in claim 1, characterised in that the next higher Regler homologue, 2-cycloalkyl-methyl-3-hydroxy-1, 4-naphthoquinone, is subjected to Hooker oxidation.

10. A composition for treating theileriosis in appropriate mammals characterised in that a compound as claimed in any one of claims 1 to 5 is presented in association with a pharmaceutically acceptable carrier.

**Revendications**

1. Composé de formule (II)

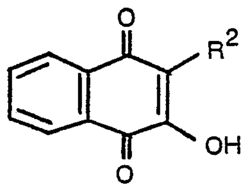

(II)

tautomère ou sel pharmaceutiquement acceptable correspondant, dans laquelle $R^2$ représente un radical cycloalcoyle monocyclique non substitué de 7 à 12 atomes de carbone.

2. Composé suivant la revendication 1, dans lequel $R^2$ compte 7 ou 8 atomes de carbone.

3. Composé suivant la revendication 1 ou 2, sous forme d'un sel de sodium, de potassium ou de N-méthylglucamine.

4. La 2-cycloheptyl-3-hydroxy-1,4-naphtoquinone.

5. La 2-cyclo-octyl-3-hydroxy-1,4-naphtoquinone.

6. Composé de formule (II) tel que défini dans l'une quelconque des revendications 1 à 5 à l'usage de la médecine vétérinaire.

7. Procédé de préparation d'un composé de formule (II) tel que défini dans la revendication 1, caractérisé en ce qu'on hydrolyse en conditions alcalines le 3-halogéno analogue correspondant du composé pour obtenir le composé 3-hydroxy-2-substitué.

8. Procédé de préparation d'un composé de formule (II) tel que défini dans la revendication 1, caractérisé en ce qu'on fait réagir un composé de formule (IV)

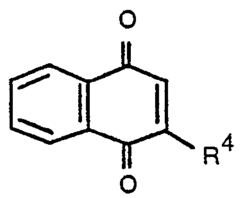

(IV)

où $R^4$ représente un radical halogéno, hydroxyle, acétoxy ou alcoxy, avec un composé donneur de radical cycloalcoyle correspondant au radical cycloalcoyle désiré $R^2$ et au cas où $R^4$ est autre qu'hydroxyle ou hydrolyse le 3-halogéno-, 3-acétoxy- ou 3-alcoxy- composé résultant dans des conditions alcalines.

9. Procédé de préparation d'un composé de formule (II) tel que défini dans la revendication 1, caractérisé en ce qu'on soumet à l'oxydation de Hooker la 2-cyclo-alcoylméthyl-3-hydroxy-1,4-naphtoquinone homologue de Regler immédiatement supérieure.

10. Composition pour le traitement de la theilériose chez les mammifères appropriés, caractérisée en ce qu'un composé suivant l'une quelconque des revendications 1 à 5 est présenté en association avec un excipient pharmaceutiquement acceptable.

**Patentansprüche**

1. Verbindung der Formel (II)

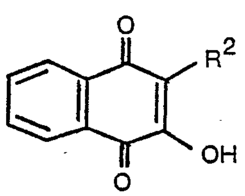

(II)

ein tautomeres oder ein pharmazeutisch verträgliches Salz davon, worin $R^2$ eine unsubstituierte monocyclische Cycloalkylgruppe mit 7 bis 12 Kohlenstoffatomen bedeutet.

2. Verbindung nach Anspruch 1, worin $R^2$ 7 oder 8 Kohlenstoffatome hat.

3. Verbindung nach Anspruch 1 oder 2 in Form eines Natrium-, Kalium- oder N-Methyl-glucaminsalzes.

4. 2-Cycloheptyl-3-hydroxy-1,4-naphthochinon.

5. 2-Cyclooctyl-3-hydroxy-1,4-naphthochinon.

6. Verbindung der Formel (II), wie in einem der Ansprüche 1 bis 5 definiert, für die Verwendung in der Veterinärmedizin.

7. Verfahren zur Herstellung einer Verbindung der Formel (II), wie sie in Anspruch 1 definiert ist, dadurch gekennzeichnet, daß das entsprechende 3-Halogen-Analoge der Verbindung unter alkalischen Bedingungen hydrolysiert wird zur Herstellung der 3-Hydroxy-2-substituierten Verbindung.

8. Verfahren zur Herstellung einer Verbindung der Formel (II), wie sie in Anspruch 1 definiert ist, dadurch gekennzeichnet, daß eine Verbindung der Formel (IV)

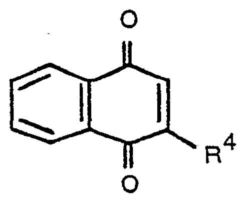

(IV)

worin $R^4$ eine Halogen-, Hydroxy-, Acetoxy- oder Alkoxy- Gruppe ist, mit einer entsprechenden Cycloalkyl-Donor-Verbindung mit der gewünschten Cycloalkylgruppe $R^2$ umgesetzt wird und dann, wenn $R^4$ eine andere Bedeutung als die von Wasserstoff hat, die resultierende 3-Halogen- oder 3-Acetoxy- oder 3-Alkoxy- Verbindung unter alkalischen Bedingungen hydrolysiert wird.

9. Verfahren zur Herstellung einer Verbindung der Formel (II), wie sie in Anspruch 1 definiert ist, dadurch gekennzeichnet, daß das nächst höhere Regler-Homologe, 2-Cycloalkyl-methyl-3-hydroxy-1,4-naphthochinon, der Hooker-Oxidation unterworfen wird.

10. Präparat zur Behandlung der Theileriose bei geeigneten Säugetieren, dadurch gekennzeichnet, daß eine Verbindung nach einem der Ansprüche 1 bis 5 in Assoziation mit einem pharmazeutisch verträglichen Träger vorliegt.